# EUROPEAN PATENT SPECIFICATION

(11) **EP 0 596 319 B1**
(45) Date of publication and mention of the grant of the patent: **10.09.1997**
(21) Application number: 93116884.3
(22) Date of filing: 19.10.1993
(51) Int. Cl.: A61N 1/368

(54) **Heart stimulator**
Vorrichtung zur Stimulation des Herzens
Appareil de stimulation cardiaque

(30) Priority: 04.11.1992 SE 9203284
(43) Date of publication of application: 11.05.1994
(73) Proprietor: Pacesetter AB, 171 95 Solna (SE)
(72) Inventor: Högnelid, Kurt, S-137 38 Västerhaninge (SE); Standberg, Hans, S-172 36 Sundbyberg (SE)
(74) Representative: Lettström, Richard Wilhelm

(56) References cited:
- CH-A- 547 644
- US-A- 4 799 486
- US-A- 4 858 610

## Description

This invention relates to a heart stimulator, comprising a pulse generator and an electrode system which contains at least one bipolar electrode with one pole arranged in the atrium and one pole in the ventricle, or at least two unipolar electrodes arranged in the atrium and ventricle respectively, for detecting atrial and ventricular electrical activity.

Such electrode systems are known through US-4.154.247 and US-4.567.901.

DDD pacemakers are currently used for treating patients with bradycardia. A conventional DDD pacemaker requires two electrodes, each with its own lead. One of the electrodes is placed in the atrium and the other in the ventricle. Electrical activity is sensed with the electrodes, and stimulation pulses are emitted when spontaneous electrical activity is absent. There has long been a wish for DDD pacemakers employing only one electrode lead, i.e. single lead DDD pacemakers. This would greatly simply implantation, compared to the implantation of two separate, basically parallel leads.

The VDD pacemaker, using only one electrode with poles in the atrium and ventricle, is one step towards a single lead DDD pacemaker design. The poles in the atrium are "floating," i.e. they are normally not in direct contact with electrically active atrial tissue. So the electrode system passes through both the atrium and ventricle. This type of VDD electrode system contains three leads in instances with a unipolar electrode in the ventricle or four leads in instances with a bipolar electrode in the ventricle.

The object of the present invention is to achieve a heart stimulator permitting detection of atrial and ventricular cardiac activity in a bipolar mode with an electrode system employing a minimum of poles, or electrodes, plus attendant leads.

This object is achieved with a heart stimulator of the above-described kind with the features specified in patent claim 1.

In a heart stimulator according to the invention, one lead therefore terminates in a pole, or electrode, placed in the atrium, and a second lead terminates in a pole, or electrode, in the ventricle. Atrial electrical activity is sensed between the atrial pole, or electrode, and the ventricular electrode, while ventricular electrical activity is measured between the pole, or electrode, in the ventricle and the stimulator housing. In principle, a bipolar system for atrial detection is obtained when measurement of atrial activity is between poles, or electrodes, in the atrium and in the ventricle. Atrial signals are often so weak that interference from muscular activity causes problems in measurements in the atrium, so bipolar electrodes have hitherto often been used in the atrium in order to minimize interference from muscles. With the system according to the invention, therefore, such a bipolar measurement system is achieved in respect to muscle interference etc. with no need for a bipolar electrode in the atrium.

Since ventricular events are also detected by the atrial measurement unit, whereas electrical atrial events are only detected by the atrial measurement unit, a logic unit is provided, according to one advantageous refinement of the heart stimulator according to the invention, which approves the signal from the atrial measurement unit as an atrial event only if the ventricular measurement unit fails to detect any signal occurring during a defined period of time around the time an atrial signal is measured.

According to an advantageous refinement of the system according to the invention, a second pole arranged in the atrium is connected, via a resistor, to the lead for the ventricular pole. This resistor is 1-20 kohms. As a result of the resistor, this pole cannot be employed for stimulation in the atrium, as resistance is lower in the ventricular pole connected to the same lead. The value of the resistor must not be too low, since the connected atrial pole would then draw current making stimulation through the ventricular pole more difficult or impossible. Even in this instance, ventricular activity is sensed over the ventricular pole, and atrial activity is sensed between the two atrial poles. So both ventricular and atrial activity is carried by the same lead. However, the atrial signal has a considerably lower amplitude than the ventricular signal because the voltage of the atrial signal is split because of the resistor. The ventricular measurement unit's sensitivity is therefore set so it detects strong ventricular signals but not weak atrial signals. Atrial activity is measured between the poles in the atrium, as noted above, and the logic unit approves the signal from the atrial measurement unit as a genuine atrial event only if no ventricular signal is concomitantly present.

The split in the voltage of the detected atrial signal depends on the magnitude of the resistor used for the second atrial pole. If e.g. a 1 kohm resistor is used, about 1/10 of the atrial signal reaches the heart stimulator. In practice, the ventricular electrode then also serves as the indifferent electrode for sensed atrial signals.

The pole, or poles, in the atrium can be "floating", i.e. not be in direct contact with electrically active tissue, if they are only to be used for detecting electrical activity. However, if the electrode system is devised so the atrial pole, which is directly connected to its lead, is in direct contact with atrial tissue, the atrial pole can also be used for stimulating the atrium. This thereby results in a single lead DDD pacemaker only using two leads in the electrode cable.

When a bipolar electrode is employed in the ventricle, three leads are required in the electrode cable.

As exemplifications, selected embodiments of the heart stimulator according to the invention will now be described in greater detail referring to the attached drawings in which
FIG. 1 shows a heart stimulator according to the invention with a bipolar electrode implanted in a heart;
FIG. 2 shows signals obtained with a version depicted in FIG. 1;
FIG. 3 shows a heart stimulator according to the invention with an implanted electrode system using two unipolar electrodes;
FIG. 4 schematically shows another embodiment of the heart stimulator according to the invention with two poles in the atrium;
FIG. 5 shows signals obtained with the implementation depicted in FIG.4;
FIG. 6 and 7 schematically show practical implementations of the embodiments in FIGS. 3 and 4 respectively; and
FIG. 8 shows the signals detected by the atrial and ventricular measurement units in experiments with animals.

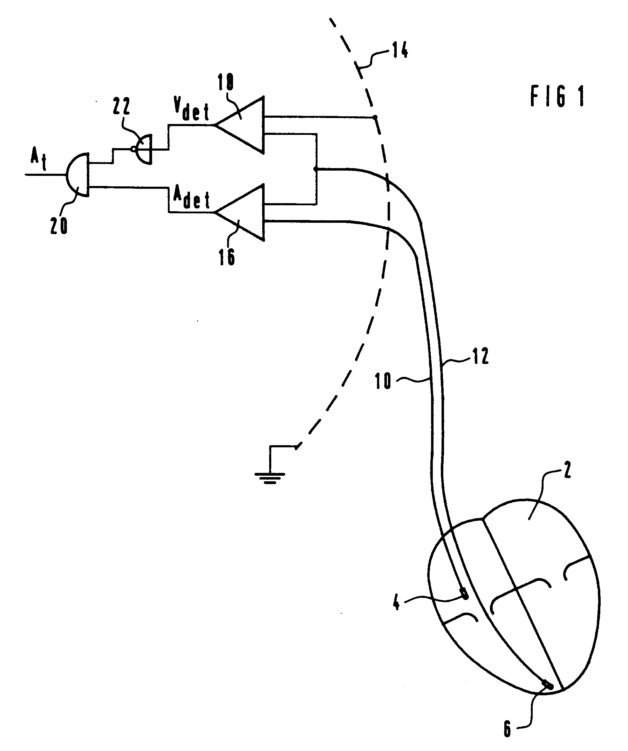

In FIG. 1 is schematically shown an electrode system according to the invention implanted in a heart 2. The electrode system contains a bipolar electrode with one pole 4 arranged in the atrium and one pole 6 arranged in the ventricle. The electrode system comprises a single electrode cable containing one lead 10, 12 each for the poles 4 and 6 respectively.

The stimulator housing is schematically indicated at 14.

The leads 10, 12 are connected to the input terminals on an atrial measurement unit 16. The lead 12 is connected in like manner to one of the input terminals on a ventricular measurement unit, one of whose input terminals is connected to the stimulator housing 14.

Thus, the atrial measurement unit 16 measures the signal between the poles 4, 6. However, the atrial measurement unit 16 detects both atrial and ventricular electrical events, since the lead 12 from the ventricular pole 6 is directly connected to the measurement unit 16. The output signal from the measurement unit 16 is connected to one of the input terminals on an AND gate 20. The output signal from the ventricular measurement unit 18 is fed, via a negating element 22, to the AND gate's 20 other input terminal. Thus, an output signal Aₜ is only obtained from the AND gate's 20 output terminal when an atrial event is detected without detection of any ventricular event around the time of the atrial detection. Here, observation of the ventricular signal must be last for a sufficiently long period of time around the time the atrial event is detected. Thus, the output signal Aₜ constitutes detection of a genuine atrial event.

The ventricular measurement unit 18 emits an output signal V_{det} which directly designates ventricular events.

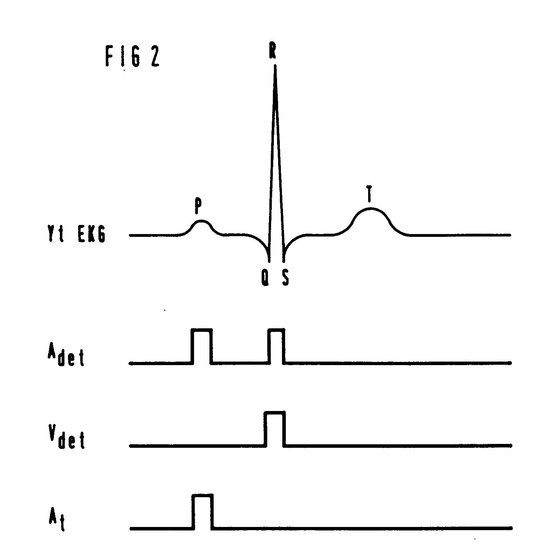

FIG. 2 shows a surface ECG, signals A_{det} and V_{det} in the form of pulses at the output terminals of the atrial and ventricular measurement units 16, 18 and the output signal Aₜ from the AND gate 20.

In the embodiment shown in FIG. 1, the atrial pole 4 is floating, i.e. it is not in direct contact with atrial tissue. So the atrial pole 4 is not suitable for stimulation and is normally only used for sensing atrial events.

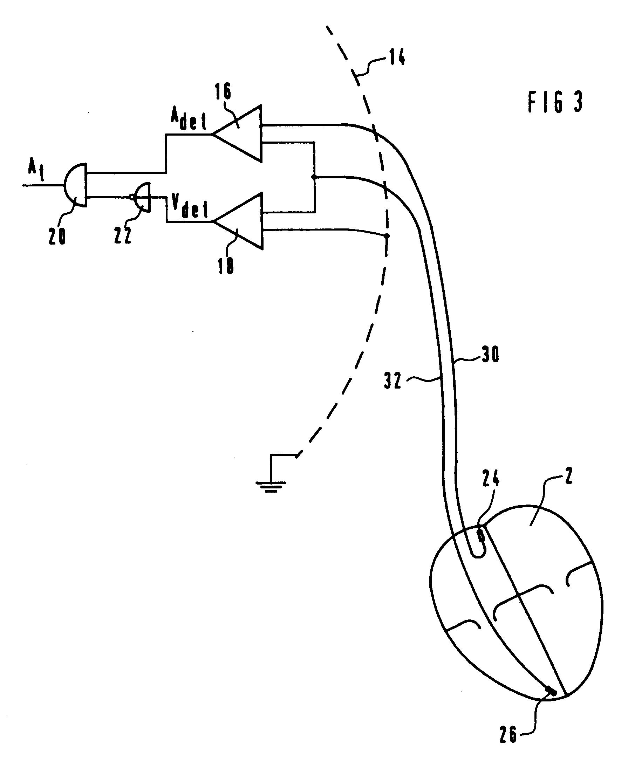

In FIG. 3 is shown an embodiment in which the electrode system contains unipolar electrodes 24, 26 in the atrium and ventricle respectively. In this instance, the atrial electrode 24 is arranged in direct contact with atrial heart tissue, making effective stimulation of the atrium is also possible. Thus, FIG. 3 depicts a DDD pacemaker with two leads 30, 32 for the unipolar electrodes 24, 26.

The sensing of atrial and ventricular events functions the same as in the embodiment shown in FIG. 1.

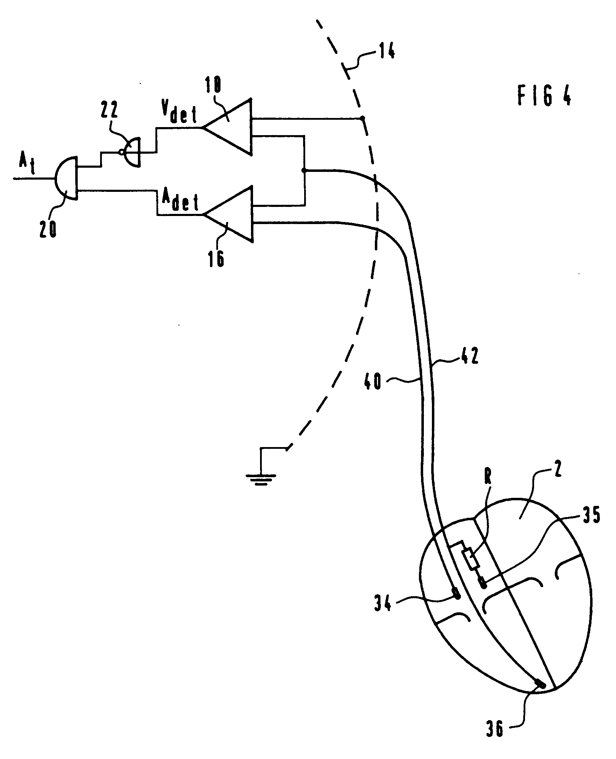

In FIG. 4 is shown another embodiment, similar to the one in FIG. 1, to which an additional atrial pole 35 is connected, via a resistor R, to the lead 42 between the heart stimulator 14 and the ventricular sensing and stimulating pole 36. The value of the resistor is 1-20 kohms, and a resistor size is selected so there is no stimulation in the atrium through the lead 42, since the pole 36 in the ventricle has a lower resistance. The atrial pole 34 could also be connected to its lead 40 via a resistor so balanced impedance is attained between the two atrial poles 34 and 35.

In this instance, detection of ventricular signals is in the same way as in the embodiments depicted in FIGS. 1 and 3. The ventricular signal is normally much stronger than the atrial signal, and the sensitivity of the ventricular measurement unit 18 is set so only the relatively strong ventricular signals are detected but not the weak atrial signals.

Detection of atrial events is made from the signal between poles 34 and 35 in the atrium. When atrial cardiac activity is present, a differential signal is obtained between poles 34 and 35 in the atrium, and this signal is therefore a measure of atrial activity. However, the atrial measurement unit 16 also detects ventricular events, since the lead 42 sensing ventricular activity is connected to the atrial measurement unit 16. So ventricular activity is detected both by the unipolar ventricular detector 18 and by the differential atrial detector 16, whereas atrial events are only detected by the atrial detector 16. For this reason, the logic unit 20, 22 is arranged to define as genuine only atrial events detected without concomitant (by some margin) ventricular detections, as described above.

The selection of atrial and ventricular events could be performed in some other way than with the logic elements 20, 22 in the above-described embodiments.

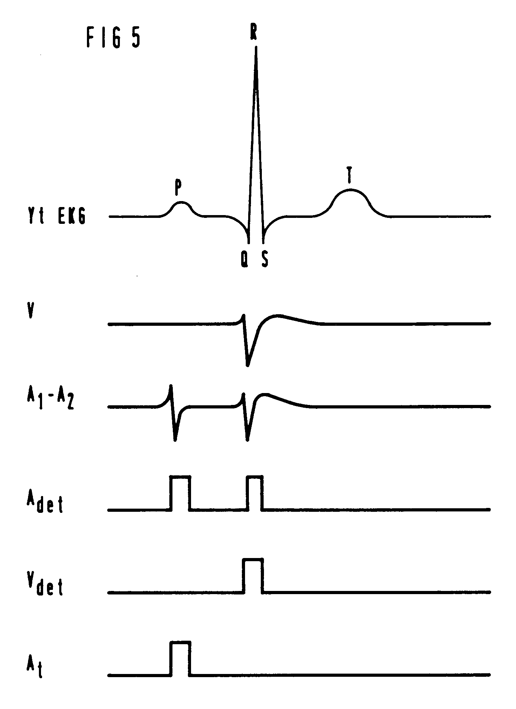

In FIG. 5 is shown the chronology of the different signals in the embodiment depicted in FIG. 4.

At the top of the FIG. is shown a conventional surface ECG. V designates the signal picked up by the ventricular pole 36, and A₁ and A₂ illustrate the differential signal between the two leads 40 and 42. A_{det} designates the output signal from the atrial measurement unit 16 and V_{det} designates the output signal from the ventricular measurement unit 18. Finally, Aₜ designates the output signal from the AND gate 20. Thus, the signal Aₜ designates "genuine" atrial events, i.e. the P wave in the ECG, and V_{det} designates "genuine" ventricular events, i.e. the QRS complex in the ECG.

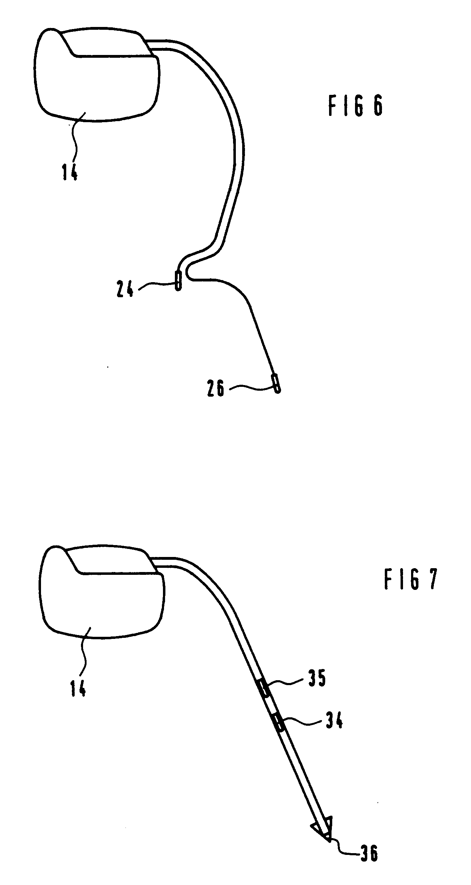

FIG. 6 schematically illustrates one practical implementation of the type of heart stimulator according to the invention, as shown in FIG. 3, wherein the electrode leads 30, 32 are bent in the atrial area so the atrial electrode 24 comes into contact with atrial tissue after the implantation.

FIG. 7 schematically illustrates the practical implementation of the embodiment shown in FIG. 4.

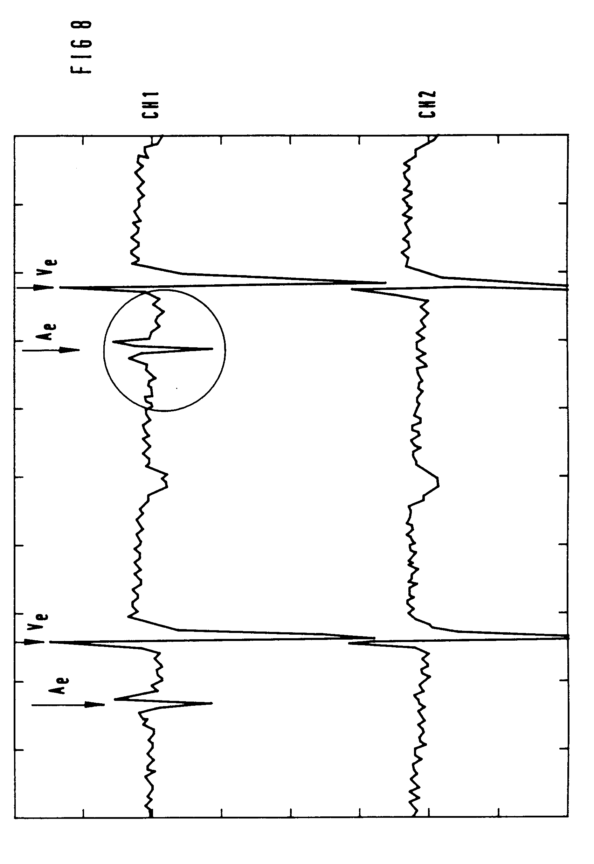

FIG. 8 shows signals picked up by the heart stimulator according to the invention in experiments on pig. The measurements were made, with a version depicted in FIG. 3, with one well-positioned electrode in the atrium and one electrode in the ventricle. The upper signal channel CH1 shows the signal measured between the atrial and ventricular poles, i.e. the atrial signal, and the second signal channel CH2 shows the signal measured between the ventricular pole and the stimulator housing, i.e. the ventricular signal. In the diagram, atrial events have been designated Aₑ and ventricular events Vₑ. As shown in the diagram, the atrial signal CH1 includes both atrial and ventricular events, whereas the ventricular signal CH2 only shows ventricular events. "Genuine" atrial events can be extracted from the measured atrial signal CH1 when the abovedescribed logical condition is applied to the atrial signal CH1.

The abovedescribed embodiments of the invention are for heart stimulators in the form of pacemakers. However, the invention can also be applied to defibrillators.

## Claims

1. A heart stimulator, comprising a pulse generator in a stimulator housing (14) and an electrode system which contains at least one bipolar electrode with one pole (4, 34) arranged in the atrium and one pole (6, 36) in the ventricle, or at least two unipolar electrodes (24, 26) arranged in the atrium and ventricle respectively, for detecting atrial and ventricular electrical activity signals, wherein an atrial measurement unit (16) is arranged to measure the signal between the two poles (4, 6, 34, 36) of the bipolar electrode, or between the two unipolar electrodes (24, 26), and a ventricular measurement unit (18) is arranged to measure the signal between the ventricular pole (6, 36), or the unipolar electrode (26) arranged in the ventricle, and the stimulator housing (14).

2. A heart stimulator of claim 1, wherein a logic unit (20, 22) is connected to the measurement units (16, 18) to approve signals from the atrial measurement unit (16) as atrial events only if the ventricular measurement (18) unit fails to detect any signal for a defined period of time around the time an atrial signal is measured.

3. A heart stimulator of claims 1 or 2 in which the electrode system comprises two leads (40, 42) from the pulse generator to the poles (34, 36) arranged in the atrium and ventricle, wherein a second pole (35) arranged in the atrium is connected to the lead (42) for the ventricular pole (36) via a resistor (R), and the atrial measurement unit (16) is connected between the two leads (40, 42).

4. A heart stimulator of any of claims 1 - 3, wherein the atrial pole(s) (4, 34, 35) is/are floating.

5. A heart stimulator of claim 1 or 2, wherein the pole (34) arranged in the atrium, or one of the poles in a bipolar electrode arranged in the atrium, or the pole in a unipolar electrode (24) arranged in the atrium, is arranged in contact with atrial tissue for electrical stimulation of same.

6. A heart stimulator of claims 3 and 5, wherein the atrial pole (34), which is directly connected to the lead (4), is arranged in contact with atrial tissue for electrical stimulation of same.

## Patentansprüche

1. Ein Herzschrittmacher mit einem Impulsgenerator in einem Schrittmachergehäuse (14) und einem Elektrodensystem, das mindestens eine bipolare Elektrode mit einem im Atrium angeordneten Pol (4, 34) und einem im Ventrikel angeordneten Pol (6, 36) oder mindestens zwei unipolare im Atrium bzw. im Ventrikel angeordnete Elektroden (24, 26) zum Detektieren atrieller und ventrikulärer elektrischer Aktivitätssignale aufweist, wobei eine atrielle Meßeinheit (16) dazu vorgesehen ist, die Signale zwischen den zwei Polen (4, 6, 34, 36) der bipolaren Elektrode oder zwischen den zwei unipolaren Elektroden (24, 26) zu messen, und wobei eine ventrikuläre Meßeinheit (18) dazu vorgesehen ist, die Signale zwischen dem ventrikulären Pol (6, 36) oder der unipolaren, im Ventrikel angeordneten Elektrode (26), und dem Schrittmachergehäuse (14) zu messen.

2. Ein Herzschrittmacher nach Anspruch 1, worin eine Logikeinheit (20, 22) an die Meßeinheiten (16, 18) angeschlossen ist, um Signale von der atriellen Meßeinheit (16) nur dann als atrielle Ereignisse zu bestätigen, wenn die ventrikuläre Meßeinheit (18) für eine bestimmte Zeitdauer um die Zeit herum, zu der ein atrielles Signal gemessen wird, kein Signal detektiert.

3. Ein Herzschrittmacher nach Anspruch 1 oder 2, in dem das Elektrodensystem zwei Leitungen (40, 42) vom dem Impulsgenerator zu den im Atrium und Ventrikel angeordneten Polen (34, 36) aufweist, wobei ein zweiter im Atrium angeordneter Pol (35) über einen Widerstand( R ) mit der Leitung (42) zu dem ventrikulären Pol (36) verbunden ist und die atrielle Meßeinheit (16) zwischen den beiden Leitungen (40, 42) angeschlossen ist.

4. Fin Herzschrittmacher nach einem der Ansprüche 1 - 3, worin der/die atrielle(n) Pol(e) (4, 34, 35)erdfrei ist/sind.

5. Ein Herzschrittmacher nach Anspruch 1 oder 2, worin der im Atrium angeordnete Pol (34) oder einer der Pole der im Atrium angeordneten bipolaren Elektrode oder der Pol einer unipolaren, im Atrium angeordneten Elektrode (24) in Kontakt mit atriellem Gewebe zur elektrischen Stimulation desselben angeordnet ist.

6. Ein Herzschrittmacher nach Anspruch 3 und 5, worin der atrielle Pol (34), der direkt mit der Leitung (4) verbunden ist, in elektrischem Kontakt mit atriellem Gewebe zur elektrischen Stimulation desselben angeordnet ist.

## Revendications

1. Stimulateur cardiaque, comportant un générateur d'impulsions dans un boîtier (14) de stimulateur et un système d'électrodes, qui comprend au moins une électrode bipolaire ayant un pôle (4,34) agencé dans l'oreillette et un pôle (6,36) dans le ventricule, ou au moins deux électrodes (24,26) monopolaires agencée dans l'oreillette et le ventricule respectivement, destiné à détecter des signaux d'activité électrique auriculaire et ventriculaire, une unité (16) de mesure d'oreillette étant agencée pour mesurer le signal entre les deux pôles (4,6,34,36) de l'électrode bipolaire, ou entre les deux électrodes (24,26) monopolaires, et une unité (18) de mesure de ventricule étant agencée pour mesurer le signal entre le pôle (6,36) ventriculaire, ou l'électrode (26) monopolaire agencée dans le ventricule, et le boîtier (14) de stimulateur.

2. Stimulateur cardiaque selon la revendication 1, dans lequel une unité (20,22) logique est reliée aux unités (16,18) de mesure pour approuver des signaux issus de l'unité (16) de mesure d'oreillette en tant que événements auriculaires uniquement si l'unité (18) de mesure ventriculaire manque de détecter tout signal pendant une période de temps définie au voisinage du moment où un signal d'oreillette est mesuré.

3. Stimulateur cardiaque selon la revendication 1 ou 2, dans lequel le système d'électrode comprend deux conducteurs (40,42) allant du générateur d'impulsion aux pôles (34,36) agencés dans l'oreillette et dans le ventricule, un second pôle (35) agencé dans l'oreillette étant relié au conducteur (42) pour le pôle (36) ventriculaire par l'intermédiaire d'une résistance (R), et l'unité (16) de mesure d'oreillette étant connectée entre les deux conducteurs (40,42).

4. Stimulateur cardiaque selon l'une quelconque des revendications 1 à 3, dans lequel le(s) pôle(s) (4,34,35) d'oreillette est/sont flottant(s).

5. Stimulateur cardiaque selon la revendication 1 ou 2, dans lequel le pôle (34) agencé dans l'oreillette, ou l'un des pôles dans une électrode bipolaire agencé dans l'oreillette, ou le pôle dans une électrode (24) monopolaire agencé dans l'oreillette, est agencé en contact avec le tissu auriculaire pour une stimulation électrique de celui-ci.

6. Stimulateur cardiaque selon les revendications 3 et 5, dans lequel le pôle (34) d'oreillette, qui est directement relié au conducteur (4), est agencé en contact avec le tissu auriculaire pour une stimulation électrique de celui-ci.
